# EUROPÄISCHE PATENTSCHRIFT

(11) **EP 0 682 668 B1**
(45) Veröffentlichungstag und Bekanntmachung des Hinweises auf die Patenterteilung: **23.04.1997**
(21) Anmeldenummer: 94905694.9
(22) Anmeldetag: 25.01.1994
(51) Int. Cl.: C07D 487/22, C08K 5/34

(54) **N-VINYLGRUPPENHALTIGE GLYKOLURILDERIVATE UND IHRE VERWENDUNG ALS LICHTSCHUTZMITTEL UND STABILISATOREN FÜR ORGANISCHES MATERIAL**
N-VINYL-GROUP-CONTAINING GLYCOL URILE DERIVATIVES AND THEIR USE AS PHOTOPROTECTIVE AGENTS AND STABILISERS FOR ORGANIC MATERIAL
DERIVES DE GLYCOLURILE CONTENANT DES GROUPES N-VINYL ET LEUR UTILISATION COMME AGENTS PHOTOPROTECTEURS ET COMME STABILISANTS POUR DES MATERIAUX ORGANIQUES

(30) Priorität: 06.02.1993 DE 4303522
(43) Veröffentlichungstag der Anmeldung: 22.11.1995
(73) Patentinhaber: BASF Aktiengesellschaft, 67063 Ludwigshafen (DE)
(72) Erfinder: KRAUSE, Alfred, D-68723 Schwetzingen (DE); AUMUELLER, Alexander, D-67435 Neustadt (DE); KORONA, Eckhard, D-67434 Neustadt (DE); TRAUTH, Hubert, D-67373 Dudenhofen (DE)
(86) Internationale Anmeldenummer: EP9400182
(87) Internationale Veröffentlichungsnummer: WO9418202

(56) Entgegenhaltungen:
- EP-A- 0 213 570
- EP-A- 0 272 590
- EP-A- 0 330 131
- US-A- 4 837 403

## Beschreibung

Die vorliegende Erfindung betrifft neue N-vinylgruppenhaltige Glykolurilderivate der allgemeinen Formel I 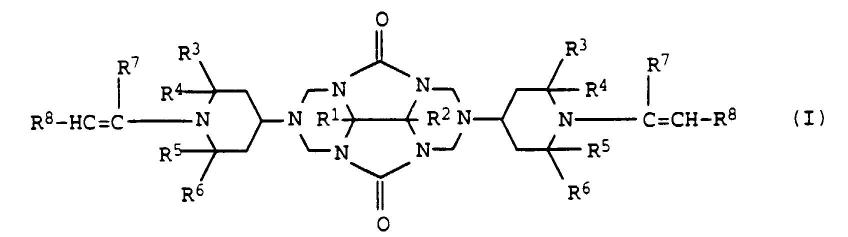 in der
- R¹ und R²: unabhängig voneinander Wasserstoff, C₁- bis C₁₂-Alkyl, C₅- bis C₈-Cycloalkyl, Phenyl, Tolyl oder C₇- bis C₁₂-Phenylalkyl bedeuten oder
- R¹ und R²: zusammen eine Tri-, Tetra- oder Pentamethylengruppierung darstellen,
- R³ bis R⁶: C₁- bis C₄-Alkyl bezeichnen,
- R⁷: für Wasserstoff, C₁- bis C₈-Alkyl, Cyano oder einen Rest der Formel COOR⁹ steht und
- R⁸: Cyano oder einen Rest der Formel COOR⁹ bedeutet, wobei
- R⁹: C₁- bis C₁₂-Alkyl, C₅- bis C₈-Cycloalkyl, Phenyl, Tolyl oder C₇- bis C₁₂-Phenylalkyl bezeichnet.

Weiterhin betrifft die Erfindung ein Verfahren zur Herstellung der Verbindungen I und ihre Verwendung als Lichtschutzmittel und Stabilisatoren für organisches Material, insbesondere für Kunststoffe und Lacke, sowie mit den Verbindungen I gegen die Einwirkung von Licht, Sauerstoff und Wärme stabilisiertes organisches Material, insbesondere stabilisierte Kunststoffe und Lacke.

Organisches Material, insbesondere Kunststoffe und Lacke, wird bekanntermaßen sehr schnell, vor allem durch Einwirkung von Licht, zerstört. Diese Zerstörung zeigt sich üblicherweise in Vergilbung, Verfärbung, Rißbildung oder Versprödung des Materials. Mit Lichtschutzmitteln und Stabilisatoren soll daher ein zufriedenstellender Schutz gegen die Zerstörung von organischem Material durch Licht, Sauerstoff und Wärme erzielt werden.

So sind beispielsweise aus der EP-B 213 570 ähnliche Glykolurilderivate wie die der vorliegenden Erfindung bekannt mit dem Unterschied, daß die dort beschriebenen Verbindungen an den Piperidin-Stickstoffatomen als Substituenten lediglich Wasserstoff, Chlor, Brom, Hydroxyl, Alkoxyl, Carboxyl, Carbonester oder gegebenenfalls substituiertes Carbamoyl aufweisen. Diese Verbindungen eignen sich ebenfalls als Stabilisatoren für organisches Material.

Derartige Mittel des Standes der Technik sind häufig noch verbesserungsbedürftig bezüglich ihrer Verträglichkeit mit Kunststoffen, der Dauer ihrer Schutzwirkung, ihrer Eigenfarbe und ihrer Neigung zur Flüchtigkeit und thermischen Zersetzung beim Einarbeiten in das zu stabilisierende Material bei erhöhter Temperatur.

Aufgabe der vorliegenden Erfindung war es daher, Lichtschutzmittel bzw. Stabilisatoren bereitzustellen, die einen noch wirkungsvolleren Schutz für organisches Material mit sich bringen.

Demgemäß wurden die eingangs definierten N-vinylgruppenhaltigen Glykolurilderivate I gefunden.

Als geradkettige oder verzweigte Alkylreste für R¹ bis R⁷ und R⁹, die als C₁- bis C₄-, C₁- bis C₈- und C₁- bis C₁₂-Alkylreste angesprochen sind, eignen sich beispielsweise Methyl, Ethyl, n-Propyl, iso-Propyl, n-Butyl, iso-Butyl, sec.-Butyl, tert.- Butyl, n-Amyl, iso-Amyl, sec.-Amyl, tert.-Amyl, Neopentyl, n-Hexyl, n-Heptyl, n-Octyl, 2-Ethylhexyl, n-Nonyl, iso-Nonyl, n-Decyl, n-Undecyl und n-Dodecyl. Bevorzugt werden generell niedere Alkylreste, vor allem C₁- bis C₄-Alkylreste, insbesondere Methyl und Ethyl.

Als C₅- bis C₈-Cycloalkylreste für R¹, R² und R⁹ kommen vor allem Cyclopentyl und Cyclohexyl, daneben auch Cycloheptyl, Cyclooctyl, Methylcyclopentyl, Dimethylcyclopentyl, Methylcyclohexyl, Ethylcyclohexyl und Dimethylcyclohexyl in Betracht.

Als C₇- bis C₁₂-Phenylalkylreste für R¹, R² und R⁹ eignen sich beispielsweise 1-Phenylethyl, 2-Phenylethyl, 1-Phenylpropyl, 2-Phenylpropyl, 3-Phenylpropyl, 2-Phenylprop-2-yl, 4-Phenylbutyl, 2,2-Dimethyl-2-phenylethyl, 5-Phenylamyl, 6-Phenylhexyl oder vor allem Benzyl.

Als Tolylreste kommen ortho-, meta und vor allem p-Tolyl in Betracht.

In einer bevorzugten Ausführungsform bedeuten die Variablen R¹ und R² Wasserstoff, Methyl, Ethyl, Phenyl, Tolyl oder Benzyl, hiervon wird Wasserstoff ganz besonders bevorzugt.

In einer weiteren bevorzugten Ausführungsform bezeichnen die Variablen R³ bis R⁶ Methyl.

In einer weiteren bevorzugten Ausführungsform steht die Variable R⁷ für Wasserstoff, Methyl, Ethyl, Carboxymethyl oder Carboxyethyl, insbesondere jedoch für Wasserstoff.

In einer weiteren bevorzugten Ausführungsform bedeutet die Variable R⁸ Carboxymethyl oder insbesondere Carboxyethyl.

Für die räumliche Stellung an der Doppelbindung von I des Restes R⁸ zum Piperidinring und zu R⁷ gibt es im Prinzip keine Einschränkungen. Es können sowohl die E- als auch die Z-Isomeren bzw. im Fall R⁷=H sowohl die cis- als auch die trans-Isomeren vorliegen. Selbstverständlich können auch Mischungen der entsprechenden cis/trans- bzw. E/Z-Isomeren vorliegen.

Die erfindungsgemäßen N-vinylgruppenhaltigen Glykolurilderivate I lassen sich in vorteilhafter Weise durch Umsetzung der entsprechenden an den Piperidin-N-Atomen unsubstituierten Glykolurilderivate der allgemeinen Formel II 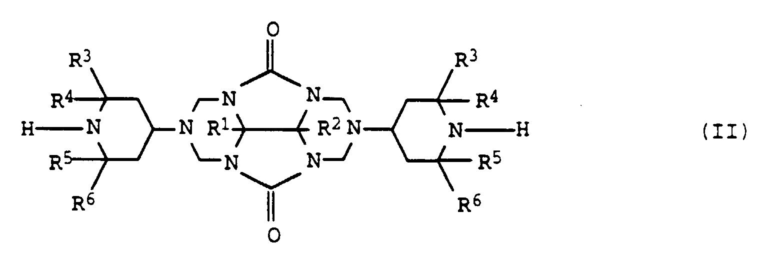 in der die Variablen R¹ bis R⁶ die obengenannte Bedeutungen haben, mit Acetylenverbindungen der allgemeinen Formel III

R⁷-C≡C-R⁸ (III)

in der die Variablen R⁷ und R⁸ die oben genannten Bedeutungen haben, in einem Lösungsmittel bei 0 bis 200°C herstellen.

Die Verbindungen II und ihre Herstellung sind aus der EP-B 213 570 bekannt.

Als Acetylenverbindungen III eignen sich beispielsweise Acetylendicarbonsäureester wie Acetylendicarbonsäuredimethyl- und -diethylester, Acetylenmonocarbonsäureester wie Propiolsäuremethyl- und -ethylester oder Cyanacetylen.

Die Verbindungen III stellen Michael-Akzeptoren dar, die sich besonders leicht an Nucleophile wie Piperidine addieren lassen.

Die Umsetzung wird zweckmäßigerweise in einem organischen Lösungsmittel, in Wasser oder in einer Mischung hieraus durchgeführt. Als organische Lösungsmittel eignen sich vor allem Kohlenwasserstoffe wie n-Hexan, Cyclohexan, Methylcyclohexan, n-Heptan, Benzol, Toluol, Xylol oder Mesitylen, Nitro- und Chlorkohlenwasserstoffe wie Methylenchlorid, Chloroform, Tetrachlorkohlenstoff, Chlorbenzol oder Nitrobenzol, Alkohole wie Methanol, Ethanol, n-Propanol, Isopropanol, n-Butanol, Isobutanol oder Butylglykol, Carbonsäureester wie Essigsäureethylester, Benzoesäuremethylester oder Butylglykolacetat sowie Amide wie Dimethylformamid, Dimethylacetamid, N-Methylpyrrolidinon oder Formamid. Als Lösungsmittel für die genannte Umsetzung kann auch mit gutem Erfolg ein Alkohol-Wasser-Gemisch, z.B. Methanol-Wasser, Ethanol-Wasser oder Isopropanol-Wasser, eingesetzt werden. Besonders bevorzugt wird Ethanol allein.

Die Umsetzung wird in der Regel bei erhöhter Temperatur, vorzugsweise bei 30 bis 180°C, insbesondere bei 50 bis 120°C, vorgenommen.

Die erfindungsgemäßen Verbindungen I eignen sich in hervorragender Weise zum Stabilisieren von organischem Material gegen die Einwirkung von Licht, Sauerstoff und Wärme. Sie sind auch wirksam als Metalldesaktivatoren. Sie werden dem zu stabilisierenden organischen Material in einer Konzentration von 0,01 bis 5 Gew.-%, vorzugsweise von 0,02 bis 2 Gew.-%, bezogen auf das organische Material, vor, während oder nach seiner Herstellung zugesetzt.

Unter organischem Material sind beispielsweise kosmetische Präparate wie Salben und Lotionen, Arzneimittelformulierungen wie Pillen und Zäpfchen, photographische Aufzeichnungsmaterialien, insbesondere photographische Emulsionen, oder Vorprodukte für Kunststoffe und Lacke, insbesondere jedoch Kunststoffe und Lacke selbst, zu verstehen.

Gegenstand der vorliegenden Erfindung ist außerdem gegen die Einwirkung von Licht, Sauerstoff und Wärme stabilisiertes organisches Material, insbesondere Kunststoffe und Lacke, welches die Verbindungen I in den oben angegebenen Konzentrationen enthält.

Zur Vermischung der erfindungsgemäßen Verbindungen I vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäßen Verbindungen I stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen I zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3, 5-Tris-(3,5-di-tert.-butyl-4-hydroxybenzyl)-isocyanurat, 1,3,5-Tris-[β-(3,5-di-tert.-butyl-4-hydroxybenzyl)-propionylethyl]-isocyanurat, 1,3,5-Tris-(2,6-dimethyl-3-hydroxy-4- tert.-butyl-benzyl)-isocyanurat und Pentaerythrit-tetrakis-[β-3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butyl-phenyl)-pentaerythritdiphosphit und Tetrakis-(2,4-di-tert.-butyl-phenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) genannt.

Weitere Antioxidantien und Lichtstabilisatoren, die zusammen mit den Verbindungen I verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Benzimidazolcarbonsäureanilide, Nickelverbindungen oder Oxalsäuredianilide.

Eine besonders gute Stabilisierung erhält man, wenn zu den Verbindungen I noch mindestens ein weiterer Lichtstabilisator aus der Verbindungsklasse der sterisch gehinderten Amine in üblicher Konzentration zugesetzt wird.

Als weitere sterisch gehinderte Amine kommen hierfür z.B. in Betracht: Bis-(2,2,6,6-tetramethylpiperidyl)-sebacat, Bis-(1,2,2,6,6-pentamethylpiperidyl)-sebacat, das Kondensationsprodukt von 1-Hydroxyethyl-2,2,6,6-tetramethyl-4-hydroxypiperidin mit Bernsteinsäure, das Kondensationsprodukt von N,N'-(2,2,6,6-tetramethylpiperidyl)-hexamethylendiamin mit 4-tert.-Octylamino-2,6-dichlor-1,3,5-triazin, Tris-(2,2,6,6-tetramethylpiperidyl)-nitrilotriacetat, Tetrakis-(2,2,6,6-tetramethyl-4-piperidyl)-1,2,3,4-butan-tetracarbonsäure, 1,1'-(1,2-Ethandiyl)-bis-(3,3,5,5-tetramethylpiperazinon), die Kondensationsprodukte von 4-Amino-2,2,6,6-tetramethylpiperidinen mit Tetramethylolacetylendiharnstoffen.

Als Kunststoffe, die durch die erfindungsgemäßen Verbindungen I stabilisiert werden können, seien beispielsweise genannt:
Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol sowie Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);
Halogenhaltige Polymere, wie z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, z.B. Polyvinylalkohol und Polyvinylacetat;
Polyurethane, Polyamide, Polyharnstoffe, Polyester, Polycarbonate, Polysulfone, Polyethersulfone und Polyetherketone.

Weiterhin können mit den erfindungsgemäßen Verbindungen I Lacküberzüge stabilisiert werden, z. B. Industrielackierungen. Unter diesen sind Einbrennlackierungen, unter diesen wiederum Fahrzeuglackierungen, vorzugsweise Zweischichtlackierungen, besonders hervorzuheben.

Die erfindungsgemäßen Verbindungen I können in fester oder gelöster Form dem Lack zugesetzt werden. Ihre gute Löslichkeit in Lacksystemen ist dabei von besonderem Vorteil.

Bevorzugt werden die erfindunsgemäßen Verbindungen I zum Stabilisieren von Polyurethanen, Polyestern, Polystyrol, Polyolefinen wie Ethylen- oder Propylenpolymerisaten, Polyamiden sowie ABS- und SAN-Polymeren, insbesondere von Formmassen hieraus, und von Lacküberzügen verwendet.

Besonders bevorzugt ist der Einsatz der erfindungsgemäßen Verbindungen I in Pulverlacken.

Die erfindungsgemäßen Verbindungen I zeichnen sich durch eine gute Verträglichkeit mit den üblichen Kunststoffarten und durch eine gute Löslichkeit und eine ausgezeichnete Verträglichkeit in den üblichen Lacksystemen aus. Sie haben in der Regel keine oder nur eine sehr geringe Eigenfarbe, sind bei den üblichen Kunststoff- und Lack-Verarbeitungstemperaturen stabil und nicht flüchtig und bewirken vor allen Dingen eine lange Schutzdauer der mit ihnen behandelten Materialien.

Der Erfindung wird durch die nachstehenden Beispiele weiter erläutert. Die Herstellbedingungen wurden nicht optimiert.

### Herstellungsbeispiel

50,2 g der Verbindung der Formel IIa (Herstellung siehe Beispiel 1 der EP-B 213 570) in 500 ml Ethanol wurden mit 26 g Propiolsäureethylester versetzt und 6 h unter Rückfluß erhitzt.

Der nach dem Abkühlen auf Raumtemperatur ausgefallene Niederschlag wurde abfiltriert, mit 100 ml n-Hexan gewaschen und bei 120°C in Wasserstrahlvakuum getrocknet. Man erhielt 59,2 g der Verbindung der Formel Ia vom Schmelzpunkt 214-216°C.

| | | |
|---|---|---|
| Ber. C 61,9 | H 8,4 | N 16,0 |
| Gef. C 61,6 | H 8,4 | N 16,0 |

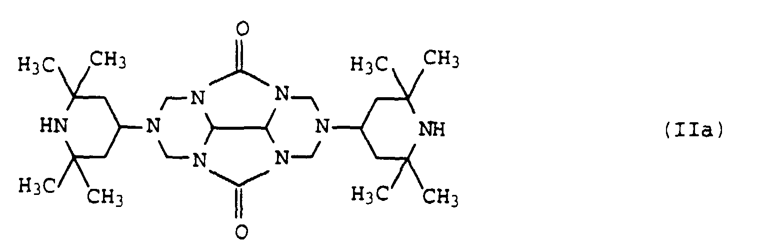

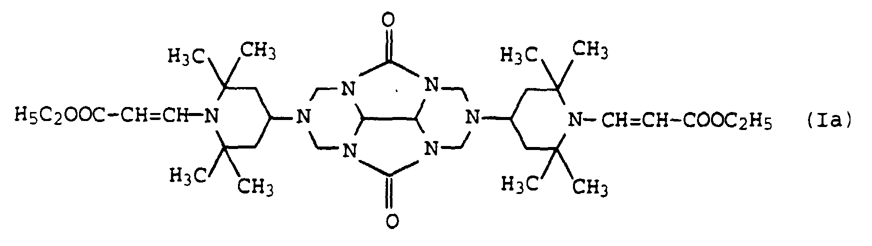

### Anwendungsbeispiel

### (a) Herstellung der Prüfkörper

980 g einer Bindemittelmischung auf Basis von hydroxylgruppenhaltigem Acrylat, 12 g der Verbindung der Formel IV (Tinuvin® 900 der Fa. Ciba-Geigy) und 8 g der erfindungsgemäßen Verbindung Ia bzw. der Vergleichssubstanz IIa wurden in einem Schnellmischer 4 min. bei 1500 Umdrehungen vorgemischt.

In einem Kneter wurde die Masse bei einer Schmelztemperatur von 90-110°C extrudiert und homogenisiert. Durch wassergekühlte Quetschwalzen wurde die Masse abgekühlt und ausgewalzt.

Das nun spröde Material wurde grob zerkleinert, mittels einer Mühle gemählen und auf einer Siebmaschine bis zu einer Korngröße ≤ 100 µm gesiebt. Durch elektrostatische Sprühbeschichtung wurde die Masse in einer Schichtdicke von 60-90 µm auf ein mit einem silbernen, wasserverdünnbaren Tönlack grundiertes Aluminiumblech appliziert und der Lack bei 180°C 20 min. eingebrannt.

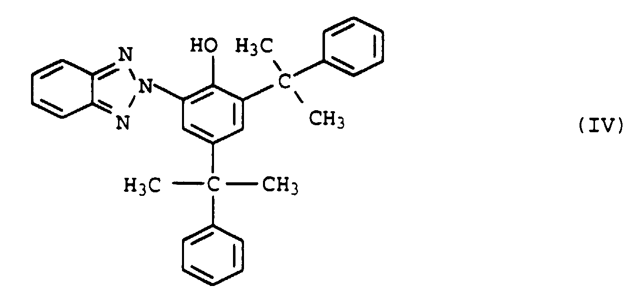

### (b) Belichtung

Die so hergestellten Proben wurden in einem Weather-O-Meter mit Kanten-Filter-A 1900 h lang bewettert. Die Beurteilung erfolgte visuell mittels Mikroskop auf Rißbildung.

### (c) Ergebnis

Die Probe mit der Verbindung Ia gemäß Herstellbeispiel zeigte keine Rißbildung, die Vergleichsprobe mit der Verbindung IIa gemäß EP-B 213 570 zeigte eine starke Rißbildung.

## Patentansprüche

1. N-vinylgruppenhaltige Glykolurilderivate der allgemeinen Formel I 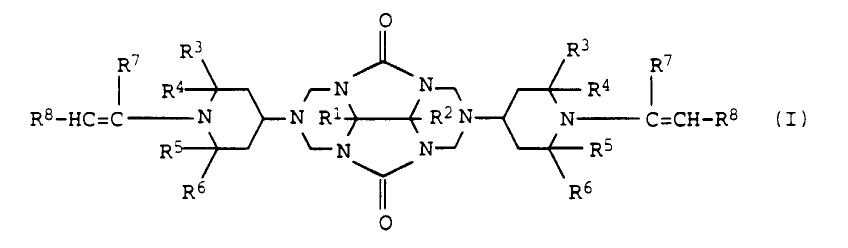 in der
R¹ und R² unabhängig voneinander Wasserstoff, C₁- bis C₁₂-Alkyl, C₅- bis C₈-Cycloalkyl, Phenyl, Tolyl oder C₇- bis C₁₂-Phenylalkyl bedeuten oder
R¹ und R² zusammen eine Tri-, Tetra- oder Pentamethylengruppierung darstellen,
R³ bis R⁶ C₁- bis C₄-Alkyl bezeichnen,
R⁷ für Wasserstoff, C₁- bis C₈-Alkyl, Cyano oder einen Rest der Formel COOR⁹ steht und
R⁸ Cyano oder einen Rest der Formel COOR⁹ bedeutet, wobei
R⁹ C₁- bis C₁₂-Alkyl, C₅- bis C₈-Cycloalkyl, Phenyl, Tolyl oder C₇- bis C₁₂-Phenylalkyl bezeichnet.

2. N-vinylgruppenhaltige Glykolurilderivate I nach Anspruch 1, bei denen R¹ und R² Wasserstoff, Methyl, Ethyl, Phenyl, Tolyl oder Benzyl bedeuten.

3. N-vinylgruppenhaltige Glykolurilderivate I nach Anspruch 1 oder 2, bei denen R³ bis R⁶ Methyl bezeichnen.

4. N-vinylgruppenhaltige Glykolurilderivate I nach den Ansprüchen 1 bis 3, bei denen R⁷ für Wasserstoff, Methyl, Ethyl, Carboxymethyl oder Carboxyethyl steht.

5. N-vinylgruppenhaltige Glykolurilderivate I nach den Ansprüchen 1 bis 4, bei denen R⁸ Carboxymethyl oder Carboxyethyl bedeutet.

6. Verfahren zur Herstellung von N-vinylgruppenhaltigen Glykolurilderivaten I gemäß den Ansprüchen 1 bis 5, dadurch gekennzeichnet, daß man Glykolurilderivate der allgemeinen Formel II 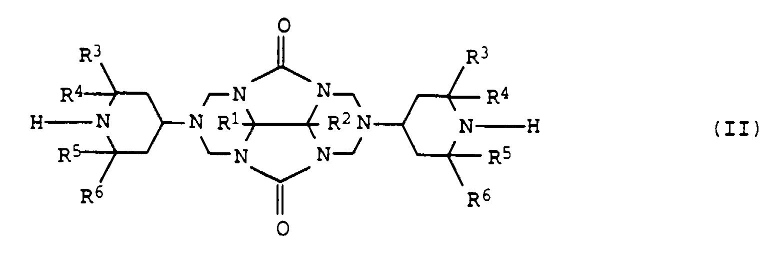 in der die Variablen R¹ bis R⁶ die oben genannten Bedeutungen haben, mit Acetylenverbindungen der allgemeinen Formel III
R⁷-C≡C-R⁸ (III)
in der die Variablen R⁷ und R⁸ die oben genannten Bedeutungen haben, in einem Lösungsmittel bei 0 bis 200°C umsetzt.

7. Verwendung von N-vinylgruppenhaltigen Glykolurilderivaten I gemäß den Ansprüchen 1 bis 5 als Lichtschutzmittel und Stabilisatoren für organisches Material.

8. Verwendung von N-vinylgruppenhaltigen Glykolurilderivaten I gemäß den Ansprüchen 1 bis 5 als Lichtschutzmittel und Stabilisatoren für Kunststoffe und Lacke.

9. Verfahren zum Stabilisieren von organischem Material gegen die Einwirkung von Licht, Sauerstoff und Wärme, dadurch gekennzeichnet, daß man hierzu N-vinylgruppenhaltige Glykolurilderivate I gemäß den Ansprüchen 1 bis 5 verwendet.

10. Verfahren zum Stabilisieren von Kunststoffen und Lacken gegen die Einwirkung von Licht, Sauerstoff und Wärme, dadurch gekennzeichnet, daß man hierzu N-vinylgruppenhaltige Glykolurilderivate I gemäß den Ansprüchen 1 bis 5 verwendet.

11. Gegen die Einwirkung von Licht, Sauerstoff und Wärme stabilisiertes organisches Material, enthaltend 0,01 bis 5 Gew.-%, bezogen auf die Menge des organischen Materials, eines oder mehrerer N-vinylgruppenhaltiger Glykolurilderivate I gemäß den Ansprüchen 1 bis 5.

12. Gegen die Einwirkung von Licht, Sauerstoff und Wärme stabilisierte Kunststoffe und Lacke, enthaltend 0,01 bis 5 Gew.-%, bezogen auf die Menge der Kunststoffe bzw. Lacke, eines oder mehrerer N-vinylgruppenhaltiger Glykolurilderivate I gemäß den Ansprüchen 1 bis 5.

## Claims

1. An N-vinyl-containing glycoluril derivative of the formula I 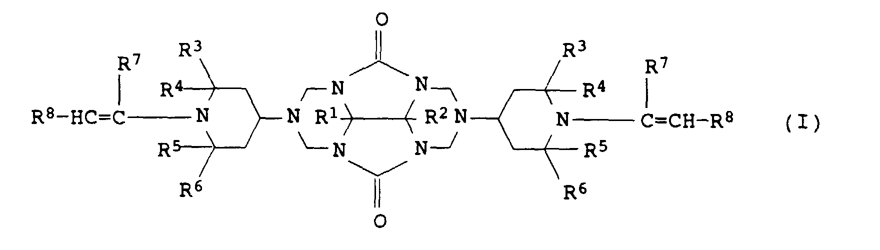 where
R¹ and R² independently of one another are each hydrogen, C₁-C₁₂-alkyl, C₅-C₈-cycloalkyl, phenyl, tolyl or C₇-C₁₂-phenylalkyl or
R¹ and R² together form a tri-, tetra- or pentamethylene group,
R³ to R⁶ are each C₁-C₄-alkyl,
R⁷ is hydrogen, C₁-C₈-alkyl, cyano or a radical of the formula COOR⁹,
R⁸ is cyano or a radical of the formula COOR⁹ and
R⁹ is C₁-Cₗ₂-alkyl, C₅-C₈-cycloalkyl, phenyl, tolyl or C₇-C₁₂-phenylalkyl.

2. An N-vinyl-containing glycoluril derivative I as claimed in claim 1, wherein R¹ and R² are each hydrogen, methyl, ethyl, phenyl, tolyl or benzyl.

3. An N-vinyl-containing glycoluril derivative I as claimed in claim 1 or 2, wherein R³ to R⁶ are each methyl.

4. An N-vinyl-containing glycoluril derivative I as claimed in any of claims 1 to 3, wherein R⁷ is hydrogen, methyl, ethyl, carboxymethyl or carboxyethyl.

5. An N-vinyl-containing glycoluril derivative I as claimed in any of claims 1 to 4, wherein R⁸ is carboxymethyl or carboxyethyl.

6. A process for the preparation of an N-vinyl-containing glycoluril derivative I as claimed in any of claims 1 to 5, wherein a glycoluril derivative of the formula II 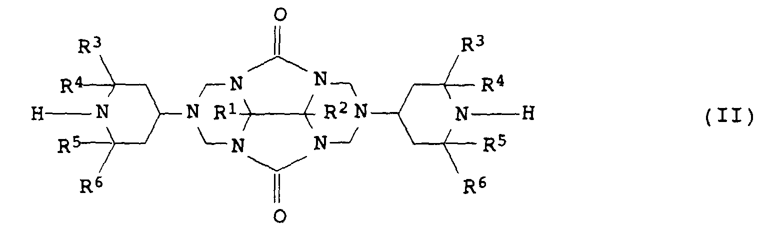 where R¹ to R⁶ have the abovementioned meanings, is reacted with an acetylene compound of the formula III
R⁷-C≡C-R⁸ (III)
where R⁷ and R⁸ have the abovementioned meanings, in a solvent at from 0 to 200°C.

7. Use of an N-vinyl-containing glycoluril derivative I as claimed in any of claims 1 to 5 as a light stabilizer or stabilizer for organic material.

8. Use of an N-vinyl-containing glycoluril derivative I as claimed in any of claims 1 to 5 as a light stabilizer or stabilizer for plastics and coatings.

9. A method for stabilizing organic material against the action of light, oxygen and heat, wherein an N-vinyl-containing glycoluril derivative I as claimed in any of claims 1 to 5 is used for this purpose.

10. A method for stabilizing plastics and coatings against the action of light, oxygen and heat, wherein an N-vinyl-containing glycoluril derivative I as claimed in any of claims 1 to 5 is used for this purpose.

11. An organic material stabilized against the action of light, oxygen and heat, containing from 0.01 to 5% by weight, based on the amount of organic material, of one or more N-vinyl-containing glycoluril derivatives I as claimed in any of claims 1 to 5.

12. A plastic or coating stabilized against the action of light, oxygen and heat, containing from 0.01 to 5% by weight, based on the amount of the plastic or coating, of one or more N-vinyl-containing glycoluril derivatives I as claimed in any of claims 1 to 5.

## Revendications

1. Dérivés de glycolurile contenant des groupes N-vinyle de formule générale I 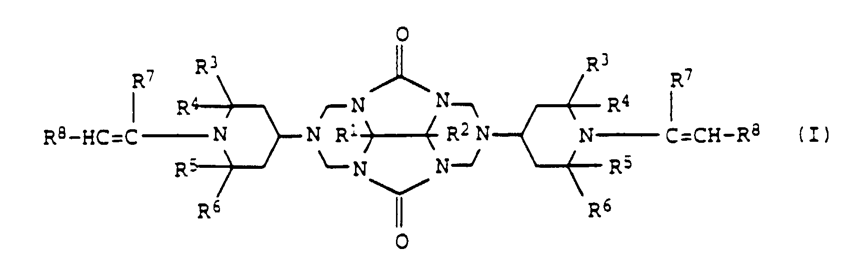 où
R¹ et R² représentent, indépendamment l'un de l'autre, un groupe alkyle en C₁ à C₁₂, cycloalkyle en C₅ à C₈, phényle, tolyle ou phénylalkyle en C₇ à C₁₂ ou
R¹ et R² désignent un groupement tri-, tétra- ou pentaméthylène,
R³ à R⁶ désignent un groupe alkyle en C₁ à C₄,
R⁷ représente un radical hydrogène, alkyle en C₁ à C₈, cyano ou un reste de formule COOR⁹ et
R⁸ désigne un radical cyano ou un reste de formule COOR⁹, tandis que
R⁹ représente un groupe alkyle en C₁ à C₁₂ cycloalkyle en C₅ à C₈, phényle, tolyle ou phénylalkyle en C₇ à C₁₂.

2. Dérivés de glycolurile contenant des groupes N-vinyle I selon la revendication 1, dans lesquels R¹ et R² représentent l'hydrogène ou un groupe méthyle, éthyle, phényle, tolyle ou benzyle.

3. Dérivés de glycolurile contenant des groupes N-vinyle I selon l'une des revendications 1 ou 2, dans lesquels R³ à R⁶ désignent un groupe méthyle.

4. Dérivés de glycolurile contenant des groupes N-vinyle I selon l'une quelconque des revendications 1 à 3, dans lesquels R⁷ désigne l'hydrogène ou un groupe méthyle, éthyle, carboxyméthyle ou carboxyéthyle.

5. Dérivés de glycolurile contenant des groupes N-vinyle I selon l'une quelconque des revendications 1 à 4, dans lesquels R⁸ représente un groupe carboxyméthyle ou carboxyéthyle.

6. Procédé pour la préparation de dérivés de glycolurile contenant des groupes N-vinyle I selon l'une quelconque des revendications 1 à 5, caractérisé par le fait qu'on fait réagir des dérivés de glycolurile de formule générale II 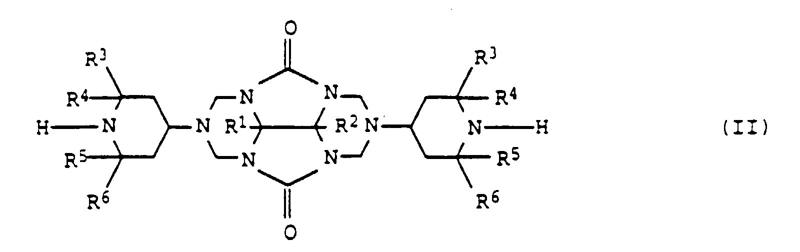 où les variables R¹ à R⁶ ont les significations susdites, avec des composés d'acétylène de formule générale III
R⁷-C≡C-R⁸ (III)
où les variables R⁷ et R⁸ ont les significations susdites, dans un solvant, entre 0 et 200°C.

7. Utilisation des dérivés de glycolurile contenant des groupes N-vinyle I selon l'une quelconque des revendications 1 à 5 comme agents de protection contre la lumière et stabilisateurs pour matière organique.

8. Utilisation des dérivés de glycolurile contenant des groupes N-vinyle I selon l'une quelconque des revendications 1 à 5 comme agents de protection contre la lumière et stabilisateurs pour matières plastiques et laques.

9. Procédé pour la stabilisation de matière organique contre l'action de la lumière, de l'oxygène et de la chaleur, caractérisé par le fait qu'on utilise pour ce faire des dérivés de glycolurile contenant des groupes N-vinyle I selon l'une quelconque des revendications 1 à 5.

10. Procédé pour la stabilisation de matières plastiques et de laques contre l'action de la lumière, de l'oxygène et de la chaleur, caractérisé par le fait qu'on utilise pour ce faire des dérivés de glycolurile contenant des groupes N-vinyle I selon l'une quelconque des revendications 1 à 5.

11. Matière organique stabilisée contre l'action de la lumière, de l'oxygène et de la chaleur, contenant 0,01 à 5 % en poids, par rapport à la quantité de matière organique, d'un ou plusieurs dérivés de glycolurile contenant des groupes N-vinyle I selon l'une quelconque des revendications 1 à 5.

12. Matières plastiques et laques stabilisées contre l'action de la lumière, de l'oxygène et de la chaleur, contenant 0,01 à 5 % en poids, par rapport à la quantité respectivement de matière plastiques ou de laques, d'un ou plusieurs dérivés de glycolurile contenant des groupes N-vinyle I selon l'une quelconque des revendications 1 à 5.
